# EUROPEAN PATENT SPECIFICATION

(11) **EP 2 224 222 B1**
(45) Date of publication and mention of the grant of the patent: **23.10.2013**
(21) Application number: 08855112.2
(22) Date of filing: 27.10.2008
(51) Int. Cl.: G01N 1/10, G01N 33/543, B01L 3/00, B01L 3/14

(54) **TEST CONTAINER, TEST PIECE, TEST KIT AND TEST METHOD**
TESTBEHÄLTER, TESTOBJEKT, TESTKIT UND TESTVERFAHREN
RÉCIPIENT DE TEST, ÉLÉMENT DE TEST, NÉCESSAIRE DE TEST ET PROCÉDÉ DE TEST

(30) Priority: 30.11.2007 JP 2007310933
(43) Date of publication of application: 01.09.2010
(73) Proprietor: Morinaga Milk Industry Co., Ltd., Minato-ku Tokyo 108-8384 (JP)
(72) Inventor: KATSUMATA, Noriko, Zama-shi Kanagawa 228-8583 (JP)
(74) Representative: Vossius & Partner
(86) International application number: PCT/JP2008/069432
(87) International publication number: WO 2009/069412

(56) References cited:
- JP-A- 10 257 881
- JP-A- 2001 116 739
- JP-A- 2006 038 700
- JP-A- 2007 093 292
- JP-T- 11 506 213
- JP-T- 2006 504 072
- JP-U- 5 052 757
- US-A- 3 849 256
- US-A1- 2003 190 745
- US-A1- 2005 232 813
- US-B1- 6 403 383

## Description

### TECHNICAL FIELD

The present invention relates to a test vessel, a test strip, and a test kit that are suitable for a test method, and a test method which employs the same. In the method, one end of the test strip is immersed in a sample fluid and the determination is carried out by observing the visible changes occurred to the test strip.
Priority is claimed on Japanese Patent Application No. 2007-310933, filed November 30,2007.

### BACKGROUND ART

Immunochromatography is a measuring method in which, when a sample fluid containing an antigen or an antibody flows over a membrane (a test strip) due to a capillary action, a color-labeled antibody that specifically reacts with the antigen or the antibody and a capture antibody form a complex with the antigen or the antibody, and the resulting coloration is visually observed.
For example, commercial reagents for pregnancy tests measure the urinary human chorionic gonadotropin (hCG), which is a glycoprotein hormone secreted from placental villous tissues after fertilization/implantation, by an immunochromatography assay, and the reagents are widely used for the diagnosis of pregnancy, threatened abortion, prognosis of ectopic pregnancy, management of trophoblastic disease, and the like.
Specifically, the commercially available products are used by dipping one end of a stick-shaped test strip in the urine collected in a urine cup or the like until the level of urine reaches the level of a line provided on the test strip, leaving the test strip to stand after 3 seconds of immersion, and observing the presence/absence of coloration in the determination region of the test strip.

In addition, an assay for detecting rotavirus antigens in stool by immunochromatography is also known. The assay is carried out, for example, by first collecting about 12 to 13 mg of stool and suspending it in a sample extraction solution, followed by stirring with a mixer for 2 minutes or more to prepare a stool suspension. Subsequently, the suspension is collected in a vessel, and a test strip is dipped into the vessel to a level so as not to exceed a predetermined stop line and then immersed in the suspension for 30 seconds. Then the test strip is pulled out of the stool suspension, and 15 minutes later, the test strip is examined for the presence/absence of a magenta line for the determination.

These assay methods use only test strips, and thus have advantages of simple and easy operation and diagnosis with little effort. However, when a pretreatment such as a suspension treatment and a filtration treatment is required while handling samples like stool and urine, operating procedures of such pretreatments may vary among different individuals which may lead to inaccurate determinations. Moreover, when there is a technical variation among different individuals for bringing a sample fluid into contact with a test strip, this can also be a cause for inaccurate determinations.

In order to solve such problems, a diagnostic kit has been proposed, in which a treatment vessel for carrying out pretreatments such as suspension, filtration, and dropping treatments on the samples, and a test strip are combined to form one set of a kit (Patent Document 1).
When using the diagnostic kit described in Patent Document 1, an operation to stir a buffer solution and a sample (for example, stool) in a treatment vessel, for example, to prepare a sample extraction solution, and an operation to add the sample extraction solution dropwise onto a test strip from the treatment vessel are required.
When manually carrying out the dropwise addition of the sample extraction solution onto the test strip, it is possible that the procedures for bringing the test strip into contact with the sample may vary among different individuals, which may lead to inaccurate determinations.

Accordingly, a product for practical use has been developed, in which a test strip itself is accommodated in a housing so that the overall configuration is formed into a cassette shape, and one part of this housing is opened (an opening) and a sample fluid prepared separately is brought into contact with the test strip by adding the sample fluid dropwise through the opening or by immersing the opening in the sample fluid (Patent Document 2).
Patent Document 3 discloses a specific diagnostic test kit which among others has a cup-like container for retaining a fluid sample to be tested and the open top end of the container is closed by a closure cap on the open end.
[Patent Document 1] Japanese Laid-Open Patent Application, No. 2005-249388
[Patent Document 2] Japanese Patent Publication No. 2786438
[Patent Document 3] US 6,403,383 B

### DISCLOSURE OF THE INVENTION

### PROBLEMS TO BE SOLVED BY THE INVENTION

However, of the above methods according to Patent Documents 1 and 2 involve a process where a sample is handled in the open air. In other words, in the abovementioned conventional methods for measuring hCG or rotavirus antigens by immunochromatography using a test strip, all the processes are carried out in the open air. It is inevitable to carry out the process where a sample extraction solution is added dropwise from a treatment vessel to a test strip in the method disclosed in the above Patent Document 1 and the process where a sample is pretreated in the method disclosed in the above Patent Document 2, in the open air.
As a result, there are various problems such as adverse effects on work environment due to the mal odor from samples, concerns among the workers over the infectious diseases, and the need for careful waste disposal. Accordingly, it is desirable to carry out the operation as quickly as possible in a closed system.

In addition, with the conventional technique, since a process for pretreating a sample and a determination process in which a sample is brought into contact with a test strip, for example, are carried out using separate instruments and vessels, it is possible that human error due to the mix-up among different instruments and vessels or the like would occur when switching from one process to another.
Furthermore, with the conventional technique, differences in the technical skills among different individuals readily affect the examination results. For example, there has been a problem of possible technical variations among different individuals for bringing a test strip into contact with a sample, which may lead to inaccurate determinations.

The present invention is made in view of the above circumstances and its object is to provide a test vessel, a test strip, a test kit, and a test method which enable the reduction of the degree of exposure of samples to the external environment as well as the execution of simpler and more accurate tests such as the examination and diagnosis of samples:

### MEANS FOR SOLVING THE PROBLEMS

In order to achieve the above object, the present invention employed the following aspects.
A first aspect is a test vessel used in a test method having a step of immersing one end of a test strip in a sample fluid, the test vessel characterized by including: a vessel body having an opening at an upper end thereof as well as a storage space communicating with the opening and in which the sample fluid is stored; a lid body that seals the opening of the vessel body in an attachable/detachable manner; and a test strip fixing section provided in the lid body which fixes the other end of the test strip in an attachable/detachable manner and holds the test strip within the storage space while sealing the opening of the vessel body with the lid body, wherein the lid body is composed of a cap fitted to the opening of the vessel body in an attachable/detachable manner and an inner packing which is formed from an elastic material and which seals a gap between the cap and the opening, and the test strip fixing section is provided in the inner packing.
A second aspect is a test vessel according to the first aspect characterized in that the test strip fixing section is a fitting hole that fits with the other end of the test strip.

A third aspect is the use of a test strip in a test method employing the test vessel of the first or second aspect and having a step of immersing one end of a test strip in a sample fluid within the vessel body, the test strip characterized by including, at the other end thereof, a laminated body where 2 or more members are laminated; and a cover sheet that continuously covers one outermost layer surface of the laminated body, an end face of the laminated body in the longitudinal direction, and the other outermost layer surface of the laminated body.

A fourth aspect is a test kit including the test vessel of the first or second aspect and the test strip of the third aspect.
A fifth aspect is a test kit according to the fourth aspect further including a filter medium that partitions the storage space of the test vessel into a plurality of spaces.

A sixth aspect is a test method employing the test vessel of the first or second aspect, the test method including a step of storing a sample fluid within the storage space of the test vessel at a predetermined amount; a step of fixing the other end of the test strip to the test strip fixing section of the lid body; and a step of sealing the opening of the test vessel with the lid body, where the test strip is fixed, and thereby immersing the other end of the test strip in the sample fluid.
A seventh aspect is a test method according to the sixth aspect in which the step of storing a sample fluid within the storage space of the test vessel includes a step of preparing the sample fluid by pretreating the sample fluid inside the test vessel.

### EFFECTS OF THE INVENTION

According to the present invention, a test vessel that enables the reduction of the degree of exposure of samples to the external environment as well as the execution of simpler and more accurate tests such as the examination and diagnosis of samples can be obtained.
The test strip according to the present invention is suitably used when combined with the test vessel of the present invention, and by using them, the reduction of the degree of exposure of samples to the external environment as well as the execution of simpler and more accurate tests such as the examination and diagnosis of samples can be achieved.
According to the test kit of the present invention, the reduction of the degree of exposure of samples to external environment as well as the execution of simpler and more accurate tests such as the examination and diagnosis of samples can be achieved.
According to the test method of the present invention, the reduction of the degree of exposure of samples to external environment as well as the execution of simpler and more accurate tests such as the examination and diagnosis of samples can be achieved.

### BRIEF DESCRIPTION OF THE DRAWINGS

FIG. 1 shows an embodiment of a test vessel of the present invention and is a front view of the vessel body.
FIG. 2 shows an embodiment of a test vessel of the present invention, and FIG. 2A is a cross sectional view of an inner packing whereas FIG. 2B is a plan view thereof.
FIG. 3 shows an embodiment of a test vessel of the present invention and is a cross sectional view of a cap.
FIG. 4 is a partially cross sectional front view showing an embodiment of a test vessel of the present invention.
FIG. 5 is a cross sectional view showing an embodiment of a test strip of the present invention.
FIG. 6 shows another embodiment of a test vessel of the present invention, and FIG. 6A is a cross sectional view of an inner packing whereas FIG. 6B is a plan view thereof.
FIG. 7 shows yet another embodiment of a test vessel of the present invention, and FIG. 7A is a cross sectional view of an inner packing whereas FIG. 7B is a plan view thereof.
FIG. 8 shows an embodiment of a test vessel of the present invention and is a cross sectional view of a vessel body where a filter medium is provided.

### DESCRIPTION OF THE REFERENCE SYMBOLS

1: Vessel body; 2: Lid body; 3/6/7: Inner packing; 4: Cap; 5: Test strip; 8: Filter medium; 11: Opening; 17: Storage space; 33/63/73: Fitting hole (test strip fixing section); 50: Gripping section (the other end); 52: Cover sheet; 54: Immersion area (one end).

### BEST MODE FOR CARRYING OUT THE INVENTION

### <First Embodiment>

### Test vessel

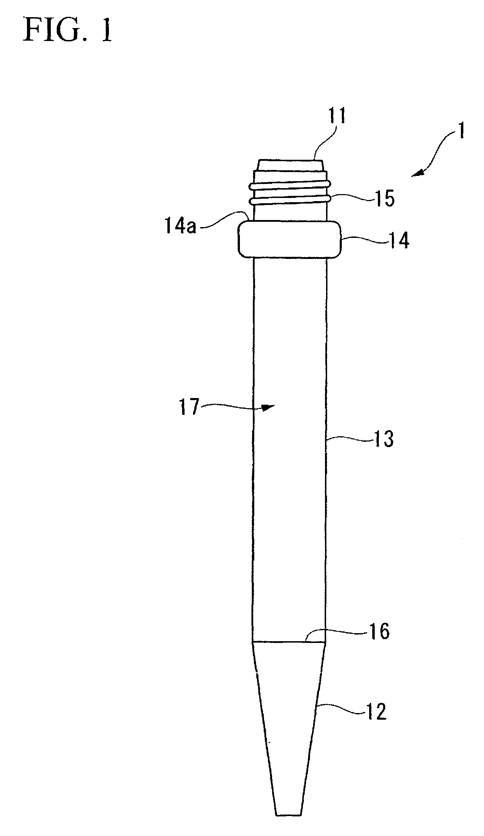

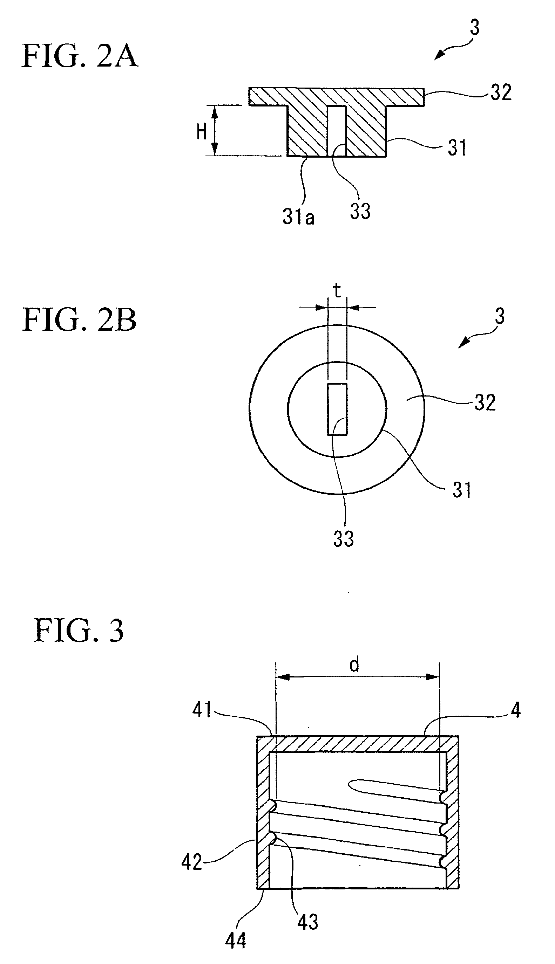

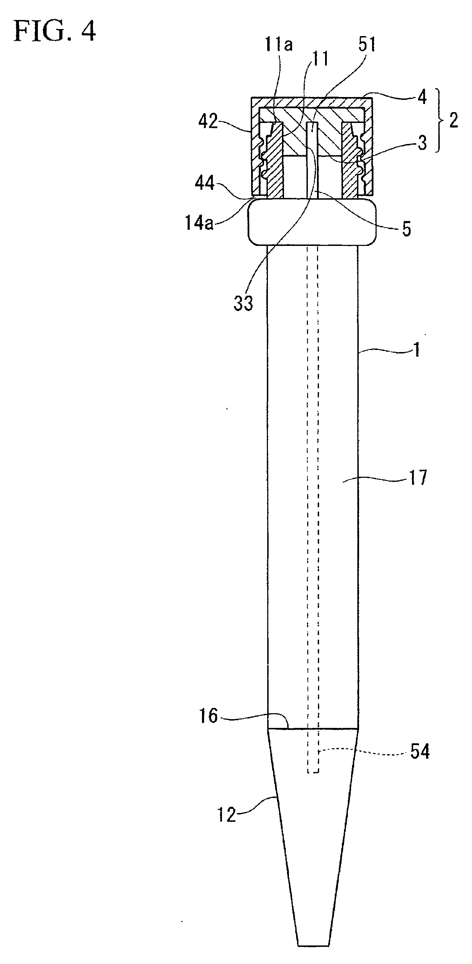

FIGS. 1 to 4 show a first embodiment of a test vessel according to the present invention and FIG. 1 is a front view of a vessel body 1. FIG. 2A is a cross sectional view of an inner packing 3 whereas FIG. 2B is a plan view of the inner packing 3 seen from the vessel body side. FIG. 3 is a cross sectional view of a cap 4. FIG. 4 is a partially cross sectional front view showing a state where a test strip 5 and a lid body 2 are set to the vessel body 1. A test vessel of the present embodiment is composed of the vessel body 1 and the lid body 2. The lid body 2 is composed of the inner packing 3 and the cap 4.

The vessel body 1 is formed of a bottomed hollow vessel having an opening 11 at its upper end. In the vessel body 1 of the present embodiment, a tapered top end section 12 that decreases in diameter towards the top end, a cylindrical barrel section 13, a swelling section 14 having an outer diameter greater than that of the barrel section 13, and a threaded section 15 to be screwed together with a threaded section 43 of the cap 4 are provided in this order from the lower end side of the vessel body.

There is no particular limitation on a material of the vessel body 1. However, it is preferable to use a synthetic resin such as polyethylene and polypropylene or a transparent material such as glass.
There is no particular limitation on the size of the vessel body 1. However, the length from the opening 11 at its upper end to its lower end is preferably about 8 to 15 cm, for example, and more preferably about 9 to 13.5 cm. The outer diameter of the barrel section 13 is preferably about 10 to 20 mm and more preferably about 12 to 18 mm.

Inside the vessel body 1 is a continuous space from the opening 11 to the end section 12 and forms a storage space 17 in which a sample fluid is stored.
In the present embodiment, an indicator line 16 is provided on the boundary between the barrel section 13 and the end section 12. The amount of stored sample fluid can be set to a predetermined constant amount by making the liquid level corresponds with the indicator line 16 when storing a sample fluid inside the vessel body 1.
In addition, scales (not illustrated) may be further provided above the indicator line 16. For example, when suspending a solid sample such as solid stool in a sample fluid after accommodating the sample fluid in the vessel until the level thereof reaches the indicator line 16, the amount of liquid increased by the addition of the solid stool (which equals the volume of the solid feces) can be easily made constant if the scales are provided.

The cap 4 is composed of a top plate section 41 and a cylindrical section 42 suspended from the circumference of the top plate section 41. The threaded section 43 that is screwed together with the threaded section 15 of the vessel body 1 is provided in the inner circumferential surface of the cylindrical section 42.
There is no particular limitation on a material of the cap 4 and known materials used in plastic-made caps can be used where appropriate.
As shown in FIG. 4, it is configured so that in a state where the cap 4 is mounted by screwing together the threaded section 15 of the vessel body 1 and the threaded section 43 of the cap 4, an end face 44 of the cylindrical section 42 abuts an upper surface 14a of the swelling section 14 of the vessel body 1, as a result of which the cap 4 is fitted to the opening 11 of the vessel body 1 in an attachable/detachable manner.
A reference symbol d in the drawing indicates the inner diameter of the thread in the threaded section 43 of the cap 4.

The inner packing 3 is composed of a disc-shaped flange section 32 and a cylinder-shaped convex section 31, The flange section 32 and the convex section 31 are concentric. The inner packing 3 is composed of an elastic material and is formed using, for example, silicone rubber or the like.
The outer diameter of the convex section 31 is formed so that it is somewhat smaller than the inner diameter of the opening 11 of the vessel body 1. As a result, it is configured so that the convex section 31 can be rotated in the direction alongside the circumference of the opening 11, while the convex section 31 is inserted inside the opening 11.
The outer diameter of the flange section 32 is formed so that it is somewhat larger than the inner diameter of the top plate section 41 of the cap 4. As a result, as shown in FIG. 4, it is configured so that the inner packing 3 can be fitted into the cap 4 by inserting, while elastically deforming, the flange section 32 into the cap 4 and closely attaching the flange section 32 to the inner surface of the top plate section 41.
A fitting hole 33 (test strip fixing section) that fits with a gripping section 50 of the test strip 5 is provided in the end face 31 a of the convex section 31. The shape, size, and depth of the fitting hole 33 is designed so that the test strip 5 does not easily fall off when the test strip 5 is inserted therein, and at the same time, the test strip 5 can be held in an attachable/detachable manner. In the present embodiment, the height H of the convex section 31 equals the depth of the fitting hole 33.

The lid body 2 according to the present embodiment is composed of the inner packing 3 and the cap 4. The inner packing 3 and the cap 4 may be separate objects and the inner packing 3 may be fitted into the cap 4 in an attachable/detachable manner in advance, or they may be integrated through bonding. In addition, the inner packing 3 and the cap 4 may be integrally formed using an elastic material.
The inner packing 3 seals a gap between the opening 11 and the cap 4. For example, as shown in FIG. 4, by putting the lid body 2, in which the inner packing 3 is integrated with the cap 4 through bonding, on the opening 11 of the vessel body 1 and screwing together the threaded section 15 of the vessel body 1 and the threaded section 43 of the cap 4, the flange section 32 of the inner packing 3 which is disposed between an end face 11a of the opening 11 and an inner surface of the top plate section 41 of the cap 4 closely attaches to the end face 11 a of the opening 11 while being elastically deformed. As a result, the opening 11 is sealed fluid-tightly. In this case, the test strip 5 fixed to the fitting hole 33 (test strip fixing section) is held within the vessel body 1 (that is, within the storage space 17) so as not to contact the inner wall.

### Test strip

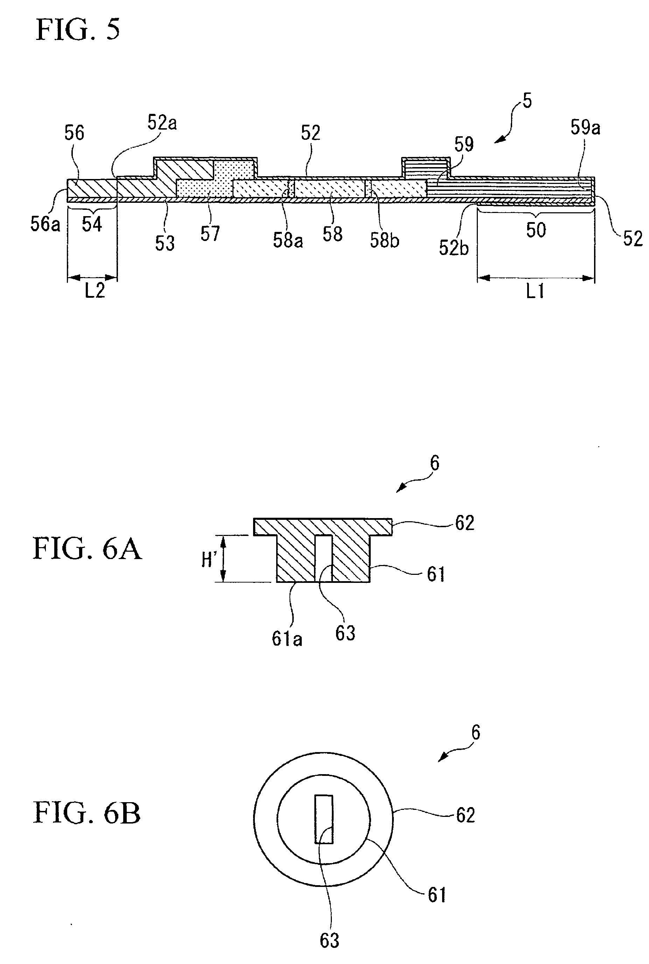

FIG. 5 is a cross sectional view schematically showing a first embodiment of the test strip 5. In the test strip 5, on a long and thin-plate shaped substrate 53, a sample absorbing section 56 having the same width as that of the substrate 53, a reaction reagent impregnating section 57, a color reaction section 58, and an absorber 59 are provided in this order along the longitudinal direction, and their upper surfaces (that is, the surfaces opposite to the substrate 53; the same applies hereinafter) are collectively covered with a cover sheet 52.
All of the sample absorbing section 56, reaction reagent impregnating section 57, color reaction section 58, and absorber 59 are constituted of materials capable of absorbing and retaining liquid, and, for example, various porous materials are used. The materials constituting the sample absorbing section 56, reaction reagent impregnating section 57, color reaction section 58, and absorber 59 may be different from each other or may be the same. For example, the sample absorbing section 56, reaction reagent impregnating section 57, and color reaction section 58 are respectively constituted with non-woven fabrics having suitable mass per unit area. The absorber 59 is preferably formed of a laminated body, in which a plurality of thin papers having a thickness of about 0.1 to 1 mm are laminated.
The cover sheet 52 and the substrate 53 are constituted of a material which barely absorbs liquid and are formed of plastic, for example. Although there is no particular limitation on a plastic material, polypropylene or the like is used, for example. There is no particular limitation on the thickness of the cover sheet 52. However, a thickness of, for example, about 0.1 to 0.5 mm is preferable, although it depends on the material. There is no particular limitation on the thickness of the substrate 53. However, a thickness of, for example, about 2 to 4 mm is preferable, although it depends on the material.

One end face 56a of the sample absorbing section 56 is flush with one end face of the substrate 53 in the longitudinal direction. The other end of the sample absorbing section 56 is laminated on one end of the reaction reagent impregnating section 57, and the other end of the reaction reagent impregnating section 57 is laminated on one end of the color reaction section 58. In addition, one end of the absorber 59 is laminated on the other end of the color reaction section 58, and the other end face 59a of the absorber 59 is flush with the other end face of the substrate 53.
An end section of the sample absorbing section 56 corresponds to an immersion area 54, and an end section of the absorber 59 corresponds to a gripping section 50.
There is no particular limitation on the length L2 of the immersion area 54. However, a length of, for example, about 5 to 20 mm is preferable.
There is no particular limitation on the length L1 of the gripping section 50. However, a length of, for example, about 5 to 40 mm is preferable.

The cover sheet 52 is formed so that it is longer than the substrate 53. The cover sheet 52 continuously and collectively covers the upper surface of the sample absorbing section 56 except the immersion area 54, the upper surface of the reaction reagent impregnating section 57, the upper surface of the color reaction section 58, and the upper surface of the absorber 59, and is bent at the edge of the absorber 59 in the longitudinal direction. The cover sheet 52 then continues to cover the end face 59a of the absorber 59 in the longitudinal direction and the end face of the substrate 53 in the longitudinal direction, bent at the edge of the substrate 53, and is laminated on the outside of the substrate 53 at a part which corresponds to the gripping section 50.
In other words, the gripping section 50 includes a laminated body, in which the substrate 53 and the absorber 59 (a laminated body of thin papers) are laminated, and the surface of one outermost layer of the laminated body, one end face of the laminated body in the longitudinal direction, and the surface of the other outermost layer of the laminated body are continuously covered by the cover sheet 52.
One end face 52a of the cover sheet 52 is positioned at a boundary with the immersion area whereas the other end face 52b thereof is positioned at a boundary with the gripping section.

Inside the test strip 5, a reaction mechanism is provided which exhibits a color reaction when reacted with a sample fluid. The reaction mechanism can employ various known reaction mechanisms. In the present embodiment, a reaction mechanism for detecting rotaviruses is provided.
In the reaction reagent impregnating section 57, a gold colloid labeled antibody (hereinafter referred to as an anti-rotavirus antibody) is impregnated, in which a mouse monoclonal antibody to rotaviruses is labeled with gold colloid in advance. Note that although an anti-rotavirus antibody is used in the present embodiment for detecting rotaviruses, when it is required to detect other pathogenic microorganisms, a primary antibody specific to the pathogenic microorganism in question is used where appropriate. In addition, although gold colloid is used for labeling the primary antibody in the present embodiment, other visualizing materials (such as colored latex particles) are also used where appropriate.
In the color reaction section 58, as the intermediate layers in the longitudinal direction, a first coloration section 58a and a second coloration section 58b are provided while being spaced from one another in the longitudinal direction. There is no particular limitation on the interval between the first coloration section 58a and the second coloration section 58b. However, it is preferably 5 mm or greater from the viewpoints of line formation and visual determination. An anti-rotavirus antibody serving as a primary antibody and an anti-mouse immunoglobulin (Ig) G antibody serving as a secondary antibody are fixed to the first coloration section 58a in the side of the immersion area 54 and the second coloration section 58b in the side of the gripping section 50, respectively. Although an anti-mouse IgG antibody is used as a secondary antibody for detecting the anti-rotavirus antibody in the present embodiment, secondary antibodies are selected appropriately depending on the type of animals producing the primary antibody to be used.
When the immersion area 54 is immersed in a sample fluid (stool sample suspension), the sample absorbing section 56 absorbs the sample fluid and the absorbed liquid moves to the reaction reagent impregnating section 57 through a capillary action. When the rotaviruses are contained in the sample fluid, they react with the gold colloid labeled antibody impregnated in the reaction reagent impregnating section 57, as a result of which a gold colloid labeled antibody-rotavirus complex is formed through an antigen-antibody reaction. This complex is further moved to the color reaction section 58 by a capillary action and is trapped by the anti-rotavirus antibody fixed to the first coloration section 58a. As a result, a magenta coloration due to the gold colloid is achieved in the first coloration section 58a. In addition, the gold colloid labeled antibody impregnated in the reaction reagent impregnating section 57 and the gold colloid labeled antibody which has not formed a complex is also transferred to the color reaction section 58. This portion of antibody is trapped by the anti-mouse IgG antibody fixed to the second coloration section after passing through the first coloration section 58a. As a result, a magenta coloration due to the gold colloid is achieved in the second coloration section 58b. Excess reagents which have not been trapped in midstream are absorbed by the absorber 59.

On the other hand, when the rotaviruses are not contained in the sample fluid, the gold colloid labeled antibody impregnated in the reaction reagent impregnating section 57 is transferred to the coloration section 58 without forming a complex, and is trapped by the anti-mouse IgG antibody fixed to the second coloration section after passing through the first coloration section 58a. As a result, a magenta coloration is achieved in the second coloration section 58b.

### [Test kit]

A test kit in the present embodiment has the test vessel (that is, the vessel body 1 and the lid body 2) and the test strip 5 according to the present embodiment. For example, the test kit is formed by accommodating the test vessel and the test strip 5 inside one outer casing or outer bag.
The respective dimensions of the vessel body 1, the lid body 2, and the test strip 5 are designed so that, as shown in FIG. 4, when the test strip 5 and the lid body 2 are set in the vessel body 1, the end face 52a of the cover sheet 52 in the immersion area 54 side is positioned at the same level as that of the indicator line 16 of the vessel body 1, and the entire immersion area 54 is positioned within the end section 12 of the vessel body 1.
For example, in the present embodiment, it is designed so that the total length of the test strip 5 including the immersion area 54 is 80 mm, the length L2 of the immersion area 54 is 15 mm, the height H of the convex section 31 of the inner packing 3 (which equals the depth of the fitting hole 33) is 5 mm, and the length of the vessel body 1 from the opening 11 at the upper end to the indicator line 16 is 65 mm.

### [Test method]

One embodiment of a test method using the test kit of the present embodiment will be described. In the present embodiment, the lid body 2 is used in which the inner packing 3 is bonded inside the cap 4 to be integrated therewith.
First, a sample fluid is stored inside the vessel body 1 (within the storage space 17) at a predetermined amount. When it is required to prepare a sample fluid by pretreating the sample, it is preferable to carry out the pretreatment inside the vessel body 1.
For example, when the sample is stool, a pretreatment for dispersing the stool in a sample extraction solution is carried out. Specifically, a sample fluid is prepared by first putting a sample extraction solution of a predetermined amount in the vessel body 1 and adding a collected sample (stool) thereto, followed by the closing of the opening 11 with the lid body 2 and the stirring of the resultant.
The collection of samples can be carried out by a known method. For example, solid stool is collected with a tip of an ear pick shaped jig. Alternatively, watery stool is collected with a jig having a swab-like tip. Subsequently, the tip of the jig is placed in the sample extraction solution inside the vessel body 1 and the solution is stirred. As a result, a solid stool is suspended or a watery stool is eluted, in the sample extraction solution. Then the jig is removed from the vessel body 1, followed by the closing of the opening 11 with the lid body 2 and the stirring of the resultant. The stirring can be carried out by using, for example, a vortex mixer.
The amount of the sample extraction solution is set so that the liquid level of the sample fluid corresponds with the indicator line 16 when the vessel body 1 is held in a manner in which the longitudinal direction thereof becomes identical to the vertical direction.

Then the lid body 2 is temporarily removed from the vessel body 1, and the test strip 5 is fixed by fitting the gripping section 50 thereof in the fitting hole 33 of the inner packing 3 of the lid body 2. At this time, the test strip 5 is inserted until the tip of the gripping section 50 thereof abuts the bottom of the fitting hole 33.
Subsequently, as shown in FIG. 4, the lid body 2 to which the test strip 5 is fixed is mounted to the opening 11 of the vessel body 1 by screwing, thereby fluid-tightly sealing the opening 11. As a result, the test strip 5 is held within the vessel body 1 (that is, within the storage space 17) without contacting the inner wall of the vessel body 1, and the entire immersion area 54 at the end thereof is immersed in a sample fluid.
Thereafter, the vessel body 1 is left to stand for a predetermined amount of time in a state where the vessel body 1 is held while its longitudinal direction becomes identical to the vertical direction. The sample fluid is absorbed inside the test strip 5 via the immersion area 54 causing a certain color reaction. The determination is made by visually observing the changes to the test strip 5 due to the color reaction, through the barrel section 13 of the vessel body 1.
In the above method, when solid matter is contained in a sample fluid, a centrifugal separation process may be carried out using a centrifugal separator after preparing the sample fluid but before immersing the test strip 5 therein.

### <Second Embodiment>

The shape of the inner packing is different between the first embodiment and the present embodiment.
FIG. 6 shows an inner packing 6 and FIG. 6A is a cross sectional view thereof whereas FIG. 6B is a plan view of the inner packing 6 seen from the vessel body side.
The inner packing 6 is composed of a disc-shaped flange section 62 and a cylinder-shaped convex section 61. The material of the inner packing 6 is the same as that in the first embodiment.
The outer diameter of the convex section 61 is formed so that it is somewhat larger than the inner diameter of the opening 11 of the vessel body 1. As a result, it is configured so that when the convex section 61 is inserted in the opening 11, the convex section 61 closely attaches to the inner wall of the opening 11 while being elastically deformed, thereby fluid-tightly sealing the opening 11 in a manner where the convex section 61 is attachable/detachable.
The outer diameter of the flange section 62 is formed so that it is greater than the outer diameter of the opening 11 and is smaller than the inner diameter d of the thread in the threaded section 43 of the cap 4. As a result, it is configured so that after sealing the opening 11 of the vessel body 1 with the inner packing 6, the cap 4 can be put thereon, and thus the threaded section 15 of the vessel body 1 and the threaded section 43 of the cap 4 can be screwed together.
In the end face 61a of the convex section 61, a fitting hole 63 (test strip fixing section) is provided as in the first embodiment. In the present embodiment, the height H' of the convex section 61 equals the depth of the fitting hole 63.

In a test method using the test vessel provided with the inner packing 6 of the present embodiment, the inner packing 6 and the cap 4 are prepared as separate objects, and the test strip 5 is fixed by fitting the gripping section 50 thereof in the fitting hole 63 of the inner packing 6. Then the inner packing 6 is inserted in the opening 11 of the vessel body 1, and after sealing the opening 11, the cap is mounted thereon. Other steps can be carried out in a similar manner to that in the first embodiment.

It should be noted that in the present embodiment, since the opening 11 of the vessel body 1 can be sealed solely with the inner packing 6, the cap 4 and the threaded section 15 of the vessel body 1 may be omitted. In other words, the lid body 2 may be composed of the inner packing 6 alone.

### [Modified Example]

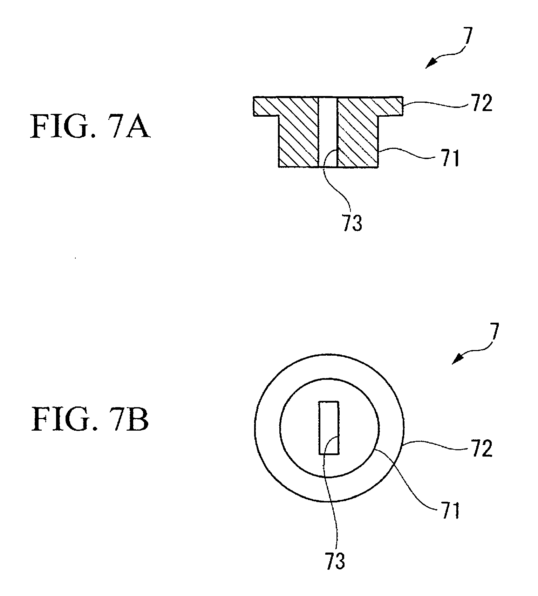

As a modified example of the second embodiment, as shown in FIG. 7, a fitting hole 73 (test strip fixing section) of an inner packing 7 may be formed so as to penetrate a convex section 71 and a flange section 72.
In the present embodiment, the gripping section 50 of the test strip 5 is inserted into the fitting hole 73 of the inner packing 7, and the inner packing 7 is inserted in the opening 11 while being elastically deformed. Thereby, the outer peripheral surface of the convex section 71 closely attaches to the inner surface of the opening 11 and the inner surface of the fitting hole 73 closely attaches to the outer surface of the gripping section 50 of the test strip 5, as a result of which the opening 11 is sealed in a fluid-tight manner.

A non-penetrating type of fitting holes like the fitting hole 63 shown in FIG. 6 is preferable in view of easy setting of the end section of the immersion area 54 of the test strip 5 at a constant position while the test strip 5 is set inside the vessel body 1.
On the other hand, a penetrating type of fitting holes like the fitting hole 73 shown in FIG. 7 is preferable in view of achieving more rigid fixation of the test strip 5.

### <Third Embodiment>

A test kit of the present embodiment includes a filter medium 8 that fits inside the vessel body 1, in addition to the test vessel according to the first or second embodiment as well as the test strip 5.

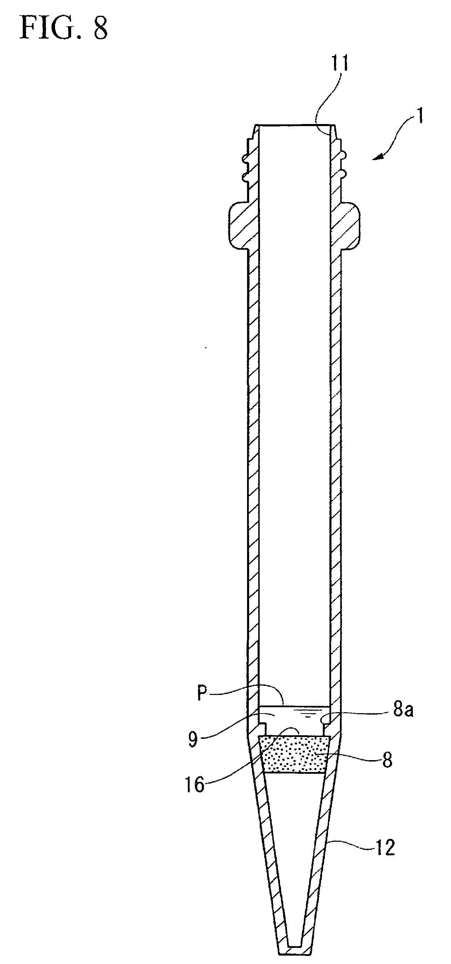

FIG. 8 is a cross sectional view showing a state where a filter medium 8 is fitted inside the vessel body 1. The illustration of the lid body 2 and the test strip 5 is omitted.
The filter medium 8 has a cylindrical shape with an outer diameter slightly greater than the inner diameter of the vessel body 1. The filter medium 8 exhibits elasticity and is constituted of a material capable of selectively permeating liquid to carry out solid-liquid separation. For example, a sponge, cotton, a non-woven fabric, or the like is used.
By inserting, while elastically deforming, the filter medium 8 inside the vessel body 1, the filter medium 8 can be fitted into the desired position in the midst of the vessel body 1 in the longitudinal direction.
There is no particular limitation on the thickness of the filter medium 8 in the longitudinal direction of the vessel body 1. However, a thickness of about 1 to 5 mm is preferable, although it depends on the material of the filter medium 8.

In the present embodiment, the filter medium 8 is fitted within an end section 12 of the vessel body 1. As a result, the storage space 17 within the vessel body 1 is partitioned into two spaces, that is, upper and lower spaces, which are separated by the filter medium 8.
In the present embodiment, a projection 8a is provided on the inner surface on the upper side of the end section 12 for preventing the filter medium 8 from moving upwards. The projection 8a may be provided continuously alongside the circumferential direction of the vessel body 1 or may be provided intermittently. There is no particular limitation on the height of the projection 8a in the radial direction of the vessel body 1. However, a height of about 0.5 to 2 mm is preferable.

One embodiment of a test method using the test kit of the present embodiment will be described. First, a sample fluid is stored inside the vessel body 1 as in the first embodiment so that the liquid level of the sample fluid corresponds with the indicator line 16.
Thereafter, the filter medium 8 is inserted in the vessel body 1 and pushed down until it reaches within the end section 12. The filter medium 8 elastically deforms to go over the projection 8a and disposed within the end section 12.
By fitting the filter medium 8 to the end section 12 filled with a sample fluid, a portion of the sample fluid permeates through the filter medium 8, as a result of which the liquid level P of the sample fluid is elevated. Since the solid matter in the sample fluid cannot permeate through the filter medium 8, a filtrate layer 9 is formed above the filter medium 8 as a result of solid-liquid separation.

Subsequently, the test strip 5 is held within the vessel body 1 as in the first embodiment, and the entire immersion area 54 of the test strip 5 is immersed in the filtrate layer 9.
In the present embodiment, the liquid level P of the filtrate layer 9 is positioned above the indicator line 16 in the first embodiment. Accordingly, by adjusting the length of the test strip 5 so that the tip of the immersion area 54 of the test strip 5 is positioned more upward than the position thereof in the first embodiment while holding the test strip 5 within the vessel body 1, the entire immersion area 54 can be immersed in the filtrate layer 9.
Thereafter, the vessel body 1 is left to stand for a predetermined time as in the first embodiment, followed by the determination due to the observation made on the visible changes to the test strip 5.

Note that in each of the above embodiments, a strip-type test paper which is commercially available and is used in immunochromatography, a common urine test paper, or the like can be used instead of the test strip 5. The above test paper may be any test paper as long as an immersion area is provided on one end thereof whereas the other end thereof can be used as a gripping section, and visible changes occur to the test paper when a test fluid penetrates into the test paper through osmosis.
Test papers with various dimensions of length, width, and thickness can be used. When changing the dimensions of a test paper, it is possible to immerse only a predetermined area at the tip of the test paper in a sample fluid by adjusting the length and thickness of a test vessel, the position of the liquid level of the sample fluid, or the like.
When using an already available test paper and the test paper has an end section serving as a gripping section, which is a laminated body where 2 or more members are laminated, it is preferable to provide a cover sheet that continuously covers one outermost layer surface of the laminated body, an end face of the laminated body in the longitudinal direction, and the other outermost layer surface of the laminated body. Due to this configuration, when inserting the gripping section in the fitting hole of the inner packing, it is easy to carry out the insertion operation while preventing the peeling off of the respective layers constituting the laminated body, and the gripping section can be stably fixed to the inner packing.

In addition, although the case where stool is used as a sample is exemplified in the above embodiment, samples are not limited thereto. Various types of samples can be used as the subject for tests as long as a sample fluid can be obtained in a liquid form. For example, urine, muddy water, hot spring water, and washing water can be used as a sample. Excrements and body fluids from humans and animals such as stool and urine are particularly suitable in the present invention.

The shape of the vessel body 1 in the cross section perpendicular to the longitudinal direction is not limited to the circular shape as in the above embodiment and can be changed to various shapes such as a triangular shape, a quadrangular shape, a polygonal shape, or an elliptical shape. In addition, the shape of the lid body 2 can be changed to various shapes in response to the cross sectional shape of the vessel body 1.

Moreover, although a fitting hole is provided in an inner packing as a test strip fixing section in the above embodiment, various fixing unit can be employed as long as it can fix the gripping section 50 of the test strip 5 in an attachable/detachable manner. For example, sandwiching unit, engaging unit, gripping unit, or the like can be employed. For stably fixing a test strip, a gripping unit involving a fitting hole or the like is preferable.

According to the above embodiment, a step of obtaining a sample fluid by pretreating the sample, a step of immersing an end section of a test paper in the sample fluid, and a step of observing visible changes occurred to the test paper can be carried out within the same test vessel. Therefore, there is no possibility of the occurrence of human errors due to the mixing-up of vessels and instruments or the like when switching from one step to another.
In addition, each of the steps can be carried out within an enclosed space. That is, if a pretreatment of samples is required, although a test vessel needs to be opened to air temporarily while fixing a test paper to a lid body after the pretreatment step, a step of immersing the test paper in a sample fluid and a step of observing visible changes can be carried out continuously inside an enclosed space without opening the test vessel in the midst of these steps.
Accordingly, safety can be improved when there is a possibility that the sample contains an infectious material or the like, and the test vessel can be discarded after completing the test as a medical waste without opening it. Alternatively, it is also possible to only discard the test strip after completing the test and to preserve the sample fluid.
In addition, when a sample is associated with mal odor, it is possible to suppress the release of the mal odor to the test environment.

Since the test strip is held inside the vessel body while being fixed to the lid body, if the shape of the test strip is constant and the position of the liquid level of the sample fluid is constant, variations from test-to-test in terms of the depth, to which the test strip is immersed in the sample fluid inside the vessel body, can be minimized. Therefore, operation of tests can be carried out simply and stably with a good reproducibility, thereby improving the accuracy of determinations.
In addition, by adjusting the length of the test strip and/or the height of the liquid level of the sample fluid, the depth to which the test strip is immersed in the sample fluid can be easily adjusted.

Since the test strip is fixed to the lid body in an attachable/detachable manner, various test strips can be adopted as long as the test strip is compatible with the vessel body and the lid body in terms of dimensions. Accordingly, a wide variety of tests can be carried out using the same test vessel. Additionally, it is also possible to readily design the vessel body and the lid body so as to suit the dimensions of various test strips. Accordingly, already available test strips can be used, which is advantageous in terms of cost.
Moreover, although the test strip is usually packaged individually in order to prevent the desiccation, the package needs to be opened just before the use of the test strip, and thus it is advantageous also from the viewpoints of preservation and protection of the test strip.

In particular, when the gripping section of the test strip is a laminated body, by providing a cover sheet that continuously covers one outermost layer surface of the laminated body, an end face of the laminated body in the longitudinal direction, and the other outermost layer surface of the laminated body, the operation for fixing the gripping section to the fitting hole of the lid body will become easy and the test strip can be fixed stably.

Although the present invention is particularly suitable for *in vitro* diagnostic products which require the carrying out of various tests and diagnosis, it is not limited to the above field.

### INDUSTRIAL APPLICABILITY

As described in detail so far, according to the present invention, a test vessel can be obtained which enables the reduction of the degree of exposure of samples to external environment as well as the execution of simpler and more accurate tests such as the examination and diagnosis of samples.
The test strip used according to the present invention is suitably used when combined with the test vessel of the present invention, and by using them, the reduction of the degree of exposure of samples to external environment as well as the execution of simpler and more accurate tests such as the examination and diagnosis of samples can be achieved.
According to the test kit of the present invention, the reduction of the degree of exposure of samples to external environment as well as the execution of simpler and more accurate tests such as the examination and diagnosis of samples can be achieved.
According to the test method of the present invention, the reduction of the degree of exposure of samples to external environment as well as the execution of simpler and more accurate tests such as the examination and diagnosis of samples can be achieved.

## Claims

1. A test vessel used in a test method having a step of immersing one end (54) of a test strip (5) in a sample fluid, the test vessel comprising:
a vessel body (1) having an opening (11) at an upper end thereof as well as a storage space (17) communicating with the opening (11) and in which the sample fluid is stored;
a lid body (2) that seals the opening (11) of the vessel body (1) in an attachable/detachable manner; and
a test strip fixing section (33, 63, 73) provided in the lid body (2) which fixes the other end (50) of the test strip (5) in an attachable/detachable manner and holds the test strip (5) within the storage space (17) while sealing the opening (11) of the vessel body (1) with the lid body (2), **characterised in that**
the lid body (2) is composed of a cap (4) fitted to the opening (11) of the vessel body (1) in an attachable/detachable manner and an inner packing (3, 6, 7) which is formed from an elastic material and which seals a gap between the cap (4) and the opening (11), and
the test strip fixing section (33, 63, 73) is provided in the inner packing (3, 6, 7).

2. The test vessel according to Claim 1, wherein the test strip fixing section (33, 63, 73) is a fitting hole that fits with the other end (50) of the test strip (5).

3. Use of a test strip (5) in a test method employing the test vessel of Claim 1 or 2 and having a step of immersing one end (54) of a test strip (5) in a sample fluid within the vessel body (1), the test strip (5) comprising, at the other end (50) thereof:
a laminated body where two or more members are laminated; and
a cover sheet (52) that continuously covers one outermost layer surface of the laminated body, an end face of the laminated body in the longitudinal direction, and the other outermost layer surface of the laminated body.

4. A test kit comprising the test vessel of Claim 1 or 2 and the test strip (5) as used in Claim 3.

5. The test kit according to Claim 4, further comprising a filter medium (8) that partitions the storage space (17) of the test vessel into a plurality of spaces.

6. A test method employing the test vessel of Claim 1 or 2, the test method comprising:
a step of storing a sample fluid within the storage space (17) of the test vessel at a predetermined amount;
a step of fixing the other end (50) of the test strip (5) to the test strip fixing section (33, 63, 73) of the lid body (2); and
a step of sealing the opening (11) of the test vessel with the lid body (2) where the test strip (5) is fixed and thereby immersing the other end (50) of the test strip (5) in the sample fluid.

7. The test method according to Claim 6,
wherein the step of storing a sample fluid within the storage space (17) of the test vessel includes a step of preparing the sample fluid by pretreating the sample fluid inside the test vessel.

## Patentansprüche

1. Ein Testbehälter, der in einem Testverfahren verwendet wird, welches einen Schritt des Eintauchens eines Endes (54) eines Teststreifens (5) in eine Probenflüssigkeit aufweist, wobei der Testbehälter umfasst:
einen Behälterkörper (1), der eine Öffnung (11) an einem oberen Ende davon sowie einen Aufbewahrungsraum (17), der mit der Öffnung (11) in Kontakt steht, aufweist und in welchem die Probenflüssigkeit aufbewahrt ist;
einen Deckelkörper (2), der die Öffnung (11) des Behälterkörpers (1) in einer anbringbaren und abnehmbaren Weise verschließt; und
einen Teststreifenbefestigungsbereich (33, 63, 73), angebracht im Deckelkörper (2), welcher das andere Ende (50) des Teststreifens (5) in einer anbringbaren und abnehmbaren Weise befestigt und den Teststreifen (5) innerhalb des Aufbewahrungsraums (17) hält, während die Öffnung (11) des Behälterkörpers (1) durch den Deckelkörper (2), verschlossen wird, **dadurch gekennzeichnet, dass**
der Deckelkörper (2) aus einer Kappe (4), welche auf die Öffnung (11) des Behälterkörpers (1) in einer anbringbaren und abnehmbaren Weise angepasst ist und einer Innenfüllung (3, 6, 7) aufgebaut ist, welche aus einem elastischen Material gebildet ist und welche eine Lücke zwischen der Kappe (4) und der Öffnung (11) verschließt, und der Teststreifenbefestigungsbereich (33, 63, 73) in der Innenfüllung (3, 6, 7) angebracht ist.

2. Der Testbehälter gemäß Anspruch 1, wobei der Teststreifenbefestigungsbereich (33, 63, 73) ein Passloch ist, auf welches das andere Ende (50) des Teststreifens (5) passt.

3. Verwendung eines Teststreifens (5) in einem Testverfahren, welches den Testbehälter gemäß Anspruch 1 oder 2 verwendet und einen Schritt des Eintauchens eines Endes (54) eines Teststreifens (5) in eine Probenflüssigkeit innerhalb des Behälterkörpers (1) aufweist, wobei der Teststreifen (5) an dem anderen Ende (50) davon umfasst:
einen laminierten Körper, wobei zwei oder mehr Bestandteile laminiert sind; und
eine Abdeckbahn (52), die kontinuierlich eine Oberfläche der äußersten Schicht des laminierten Körpers, eine Endfläche des laminierten Körpers in der Längsrichtung und die andere Oberfläche der äußersten Schicht des laminierten Körpers bedeckt.

4. Ein Testkit, das den Testbehälter gemäß Anspruch 1 oder 2 und den Teststreifen (5) wie in Anspruch 3 verwendet, umfasst.

5. Das Testkit gemäß Anspruch 4, das ferner ein Filtermedium (8), welches den Aufbewahrungsraum (17) des Testbehälters in mehrere Räume aufteilt, umfasst.

6. Ein Testverfahren, das den Testbehälter gemäß Anspruch 1 oder 2 verwendet, wobei das Testverfahren umfasst:
einen Schritt des Speicherns einer Probenflüssigkeit in dem Aufbewahrungsraum (17) des Testbehälters in einer vorbestimmten Menge;
einen Schritt des Befestigens des anderen Endes (50) des Teststreifens (5) an dem Teststreifenbefestigungsbereich (33, 63, 73) des Deckelkörpers (2); und
einen Schritt des Verschließens der Öffnung (11) des Testbehälters mit dem Deckelkörper (2), wobei der Teststreifen (5) befestigt wird und dabei Eintauchen des anderen Endes (50) des Teststreifens (5) in die Probenflüssigkeit.

7. Das Testverfahren gemäß Anspruch 6,
wobei der Schritt des Aufbewahrens einer Probenflüssigkeit in dem Aufbewahrungsraum (17) des Testbehälters einen Schritt des Herstellens der Probenflüssigkeit durch Vorbehandeln der Probenflüssigkeit in dem Testbehälter enthält.

## Revendications

1. Récipient de test utilisé lors d'un procédé de test ayant une étape consistant à immerger une extrémité (54) d'une bande de test (5) dans un fluide d'échantillon, le récipient de test comprenant :
un corps de récipient (1) ayant une ouverture (11) au niveau de son extrémité supérieure ainsi qu'un espace de stockage (17) communiquant avec l'ouverture (11) et dans lequel le fluide d'échantillon est stocké ;
un corps de couvercle (2) qui ferme hermétiquement l'ouverture (11) du corps de récipient (1) d'une manière pouvant être fixée/détachée ; et
une section de fixation de bande de test (33, 63, 73) prévue dans le corps de couvercle (2) qui fixe l'autre extrémité (50) de la bande de test (5) d'une manière pouvant être fixée/détachée et maintient la bande de test (5) à l'intérieur de l'espace de stockage (17) tout en fermant hermétiquement l'ouverture (11) du corps de récipient (1) avec le corps de couvercle (2),
**caractérisé en ce que**
le corps de couvercle (2) est composé d'un capuchon (4) monté sur l'ouverture (11) du corps de récipient (1) d'une manière pouvant être fixée/détachée et une garniture interne (3, 6, 7) qui est formée avec un matériau élastique et qui ferme hermétiquement un espace entre le capuchon (4) et l'ouverture (11), et
la section de fixation de bande de test (33, 63, 73) est prévue dans la garniture interne (3, 6, 7).

2. Récipient de test selon la revendication 1, dans lequel la section de fixation de bande de test (33, 63, 73) est un trou de raccord qui se raccorde avec l'autre extrémité (50) de la bande de test (5).

3. Utilisation d'une bande de test (5) lors d'un procédé de test utilisant le récipient de test selon la revendication 1 ou 2 et ayant une étape consistant à immerger une extrémité (54) d'une bande de test (5) dans un fluide d'échantillon à l'intérieur du corps de récipient (1), la bande de test (5) comprenant, au niveau de son autre extrémité (50) :
un corps stratifié où deux éléments ou plus sont stratifiés ; et
une feuille de couvercle (52) qui recouvre de manière continue une surface de couche située le plus à l'extérieur du corps stratifié, une face d'extrémité du corps stratifié dans la direction longitudinale, et l'autre surface de couche située le plus à l'extérieur du corps stratifié.

4. Nécessaire de test comprenant le récipient de test selon la revendication 1 ou 2, et la bande de test (5) telle qu'utilisée dans la revendication 3.

5. Nécessaire de test selon la revendication 4, comprenant en outre un milieu filtrant (8) qui divise l'espace de stockage (17) du récipient de test en une pluralité d'espaces.

6. Procédé de test utilisant le récipient de test selon la revendication 1 ou 2, le procédé de test comprenant :
une étape consistant à stocker un fluide d'échantillon à l'intérieur de l'espace de stockage (17) du récipient de test selon une quantité prédéterminée ;
une étape consistant à fixer l'autre extrémité (50) de la bande de test (5) sur la section de fixation de bande de test (33, 63, 73) du corps de couvercle (2) ; et
une étape consistant à fermer hermétiquement l'ouverture (11) du récipient de test avec le corps de couvercle (2) où la bande de test (5) est fixée et immerger ainsi l'autre extrémité (50) de la bande de test (5) dans le fluide d'échantillon.

7. Procédé de test selon la revendication 6,
dans lequel l'étape consistant à stocker un fluide d'échantillon à l'intérieur de l'espace de stockage (17) du récipient de test comprend une étape consistant à préparer le fluide d'échantillon en prétraitant le fluide d'échantillon à l'intérieur du récipient de test.
